(19) Europäisches Patentamt
European Patent Office
Office européen des brevets

(11) Veröffentlichungsnummer: **0 134 576**
**B1**

(12) # EUROPÄISCHE PATENTSCHRIFT

(45) Veröffentlichungstag der Patentschrift: **06.04.88**

(21) Anmeldenummer: **84110417.7**

(22) Anmeldetag: **01.09.84**

(51) Int. Cl.⁴: **C 07 C 43/205**, C 07 C 69/75, C 07 C 149/30, C 07 C 147/06, C 09 K 19/06

(54) **Carbocyclische Sechsringverbindungen.**

(30) Priorität: **13.09.83 DE 3332955**

(43) Veröffentlichungstag der Anmeldung: **20.03.85 Patentblatt 85/12**

(45) Bekanntmachung des Hinweises auf die Patenterteilung: **06.04.88 Patentblatt 88/14**

(84) Benannte Vertragsstaaten: **CH DE GB LI**

(56) Entgegenhaltungen:
**EP-A-0 030 879**
**DE-B-2 419 011**
**US-A-3 444 237**
**US-A-3 888 909**

**CHEMIKER-ZEITUNG, Band 107, Nr. 7/8, August 1983, Heidelberg, DE; Von WADI POULES et al.: "Hexakis(alkylsulfono)benzole, erste Schwefelhaltige discotische Flüssigkristalle"**
**THE JOURNAL OF ORGANIC CHEMISTRY, Band 46, Nr. 15, 1981, Seiten 3070-3073, Columbus, Ohio, US; F. MAIOLO et al.: "Fragmentation of aryl alkyl sulfides. A simple, one-pot synthesis of polymercaptobenzenes from polychlorobenzenes"**
**D.Demus, H. Zaschke "Flüssige Kristalle in Tabellen II", Deutscher Verlag für Grundstoffindustrie, Leipzig**

(73) Patentinhaber: **Merck Patent Gesellschaft mit beschränkter Haftung, Frankfurter Strasse 250, D-6100 Darmstadt (DE)**

(72) Erfinder: **Praefcke, Klaus, Prof. Dr., Kranzallee 62, D-1000 Berlin 19 (DE)**
Erfinder: **Kohne, Bernd, Dr., Waldstrasse 36, D-1000 Berlin 52 (DE)**
Erfinder: **Poules, Wadi, Wiesbadener Strasse 2, D-1000 Berlin 41 (DE)**
Erfinder: **Eidenschink, Rudolf, Dr., Kornblumenstrasse 1, D-6115 Münster (DE)**
Erfinder: **Scheuble, Bernhard, Dr., Am Grenzweg 18, D-6146 Alsbach- Hähnlein (DE)**

(56) Entgegenhaltungen: (Fortsetzung)
**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft Sachsringverbindungen der Formel I

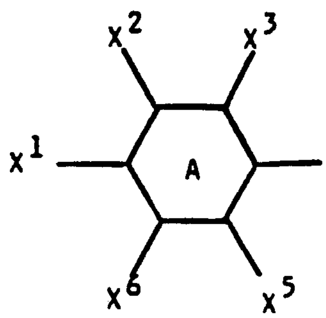

I

worin

A ein Benzol- oder Cyclohexanring ist,

$X^1$ bis $X^6$ jeweils unabhängig voneinander H, -SOR, $-SO_2R$ oder, falls A ein Cyclohexanring ist, auch -OR oder -O-COR, bedeuten, und

R jeweils eine Alkylgruppe mit bis zu 15 C-Atomen, worin eine oder zwei $CH_2$ -Gruppen durch O-Atome ersetzt sein können, ist,

wobei mindestens drei der Substituenten

$X^1$ bis $X^6$ von H verschieden sind, und, Falls A einen Cyclohexanring bedeutet, die jeweils gegenüberliegenden Substituenten equatorial und in trans-Stellung zueinander stehen.

Der Einfachheit halber bedeuten im folgenden "Ph" den Benzolkern mit sechs freien Valenzen "Cy"eine Cyclohexan-1,2,3,4,5,6-hexaylgruppe.

Ähnliche Verbindungen, nämlich Hexaalkanoyloxybenzole (entsprechend Formel I, A = Benzolring, $X^1$ bis $X^6$ = -O-COR) sind bekannt (vgl. S. Ehandrasekhar, Mol. Cryst. Liq. Cryst. 63. (1981) 171-179 und die dort angegebene Literatur); sie zeigen diskotische Eigenschaften, haben aber keine sehr breiten diskotischen Bereiche, weitere ähnliche Verbindungen sind z. B. beschrieben in J. Org. Chem., 46/15, 3070-3073 (1981); DE-B-2 419 011; US-A-3 444 237; US-A-3 888 909.

Die Verbindungen der Formel I können wie ähnliche Verbindungen als Komponenten diskotischer flüssigkristelliner Phasen verwendet werden, insbesondere für Displays, die auf dem Guest-Host-Effekt, dem Effekt der Deformation aufgerichteter Phasen, dem Effekt der dynamischen Streuung oder auf einer Anderung der Elliptisierung des Lichtes beruhen.

Der Erfindung lag die Aufgabe zugrunde, neue stabile flüssigkristalline oder mesogen Verbindungen aufzufinden, die als Komponenten dislotischer flüssigkristalliner Phasen geeignet sind. Diese Aufgabe wurde durch die Bereitstellung der Verbindungen der Formel I gelöst.

Es wurde gefunden, daß die Verbindungen der Formel I als Komponenten diskotischer flüssigkristalliner Phasenvorzüglich geeignet sind. Insbesondere sind mit ihrer Hilfe stabile diskotische flüssigkristalline Phasen mit einem breiten und für elektrooptische Effekte günstig gelegenen Temperaturbereich der Mesophase herstellbar.

Die Verbindungen der Formel I eignen sich weiterhin als anisotrope diskotische Matrix für spektroskopische Untersuchungen.

Andere Verbindungen mit diskotiachen Eigenschaften und ihre Verwendung aind beispielsweise in der US-PS-4 333 709 beschrieben.

Überraschenderweise erwiesen aich die Verbindungen der Formel I als diskotische flüssigkriatalline Verbindungen mit teilweise sehr breiten Mesobereichen.

Mit der Bereitstellung der Verbindungen der Formel wird außerdem ganz allgemein die Pelette der flüssigkristallinen Substanzen, die sich unter verschiedenen anwendungstechnischen Gesichtspunkten zur Herstellung diskotischer Gemische eignen, erheblich verbreitert.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, daß man zur Herstellung von Ethern der Formel I, worin mindestens einer der Reste $X^1$ bis $X^6$ -OR bedeutet, eine entsprechende Hydroxyverbindung verethert

und/oder daß man zur Herstellung von Estern der Formel worin mindestens einer der Reste $X^1$ bis $X^6$ -O-COR bedeutet, eine entsprechende Carbonsäure verestert

und/oder daß man zur Herstellung von Sulfoxiden der Formel I, worin mindestens einer der Reste $X^1$ bis $X^6$ -SOR bedeutet, einen entsprechenden Thioether oxidiert

und/oder daß man zur Herstellung von Sulfonen der Formel I, worin mindestens einer der Reste $X^1$ bis $X^6$ $-SO_2R$ bedeutet, einen entsprechenden Thioether oder ein entsprechendes Sulfoxid oxydiert.

Weiterhin ist Gegenstand der Erfindung die Verwendung der Verbindungen der Formel I als Komponenten diskotischer flüssigkristalliner Phasen Gegenstand der Erfindung sind ferner diskotische flüssigkristalline Phasen mit einem Gehalt an mindestens einer Verbindung der Formel I sowie Flussigkristall-Anzeigeelemente, die derartige Phasen enthelten.

Vor- und nachstehend haben A, $X^1$ bis $X^6$ und R die angegebene Bedeutung, sofern nicht ausdrücklich etwas anderes vermerkt ist.

Die Verbindungen der Formel I umfassen dementsprechend Benzolderivate der Teilformel Ia und Cyclohexanderivate der Teilformel Ib

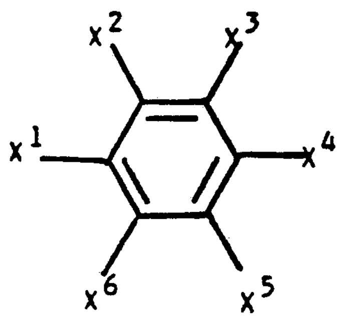

Ia

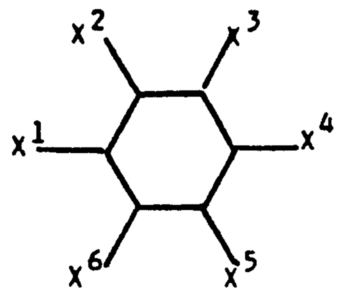

Ib

Bevorzugte Verbindungen der Formel I, in denen drei der Reste $X^1$ bis $X^6$ H bedeuten, sind die 1,3,5-trisubstituierten Verbindungen.

Bevorzugte Verbindungen der Formel I sind diejenigen, worin A ein Cyclohexanring ist.

Bevorzugte Verbindungen der Formel I sind diejenigen, in denen keiner der Reste $X^1$ bis $X^6$ H bedeutet, insbesondere diejenigen, in denen die Reste $X^1$ bis $X^6$ identisch sind und die den folgenden Teilformeln Ic bis Ih entsprechen

| | | | |
|---|---|---|---|
| | | $Cy(OR))_6$ | Ie |
| $Ph(SOR)_6$ | Ic | $Cy(SOR)_6$ | If |
| $Ph(SO_2R)_6$ | Id | $Cy(SO_2R)_6$ | Ig |
| | | $Cy(O\text{-}COR)_6$ | Ih |

Die Verbindungen der Formeln Id, Ig und Ih sind bevorzugt.

Im übrigen den Cyclohexanen der Formeln Ib sowie Ie bis Ih die jeweils gegenüberliegenden Substituenten equatorial und in trans-Stellung zueinander. Das entspricht der Konfiguration des scyllo-Inosits.

Verbindungen der Formel I, die ein oder mehrere asymmetrische C-Atome aufweisen, können in racemischer oder in optisch-aktiver Form vorliegen, wobei beide Formen von Formel I erfaßt sind.

R bedeutet einen Alkylrest, in dem auch eine (Oxaalkyl) oder zwei (Dioxaalkyl) CH -Gruppen durch O-Atome ersetzt sein können. Diese Reste können geradkettig oder verzweigt sein. Vorzugsweise sind sie geradkettig, haben 3,4, 5,6, 7, 8, 9, 10, 11, 12 oder 13 C-Atome und bedeuten demnach bevorzugt Propyl, Butyl, Pentyl, Hexyl, Heptyl, Octyl, Nonyl, Decyl, Undecyl, Dodecyl, Tridecyl, 2-Oxapropyl (= Methoxymethyl), 2- (= Ethoxymethyl) oder 3-Oxabutyl (= 2-Methoxyethyl), 2-, 3-oder 4-Oxapentyl, 2-, 3-, 4- oder 5-Oxahexyl, 2-, 3-, 4-, 5- oder 6-Oxaheptyl, ferner Ethyl, Tetradecyl, Pentadecyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 2-, 3-, 4-, 5-, 6-, 7-, 8-, 9- oder 10-Oxaundecyl, 2-, 3-, 4-, 5-, 6-, 7-, 8-, 9-, 10- oder 11-Oxadodecyl, 2-, 3-, 4-, 5-, 6-, 7-, 8-, 9-, 10-, 11- oder 12-Oxatridecyl, 2,4-Dioxapentyl, 2,4-, 2,5- oder 3,5-Dioxahexyl, 2,4-, 2,5-, 2,6-, 3,5-, 3,6- oder 4,6-Dioxaheptyl.

Verbindungen der Formeln I sowie Ia bis Ih mit verzweigten Gruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Oxa-3-methylbutyl, 3-Oxa-4-methylpentyl.

Unter den Verbindungen der Formeln 1 sowie Ia bis Ih sind diejenigen bevorzugt, in denen der Rest R eine der angegebenen bevorzugten Bedeutungen hat.

Die Verbindungen der Formel I werden nach an sich bekannten Methoden hergestellt, wie sie in der Literatur (z. B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe sind entweder bekannt, oder sie können ohne Schwierigkeiten nach an sich bekannten Methoden in Analogie zu bekannten Verbindungen hergestellt werden. Sie können gewünschtenfalls auch in situ gebildet werden, derart, daß men sie aus dem Reaktionsgemisch nicht isoliert, sondern sofort weiter zu den Verbindungen der Formel I umsetzt.

Ether der Formel I (worin mindestens einer der Reste $X^1$ bis $X^6$ OR bedeutet) sind durch Vertherung entsprechender Hydroxyverbindungen erhältlich, wobei die Hydroxyverbindung zweckmäßig zunächst in ein entsprechendes Metallderivat, z. B. durch Behandeln mit NaH, $NaNH_2$, NaOH, KOH, $Na_3CO_3$ oder $K_2CO_3$ in das entsprechende Alkalimetallalkoholat oder Alkalimetallphenolat übergeführt wird. Dieses kann dann mit dem entsprechenden Alkylhelogenid, -sulfonat oder Dialkylsulfat umgesetzt werden, zweckmäßig in einem inerten Lösungsmittel wie Aceton, 1,2-Dimethoxyethan, Dimethylformamid (DMF) oder Dimethylsulfuxid oder auch einem Überschuß an wäßriger oder wäßrig-alkoholischer NaOH oder KOH bei Tempereturen zwischen etwa 20° und 100°.

Ester der Formel I, worin mindestens einer der Reste $X^1$ bis $X^6$ -O-COR bedeutet, sind erhältlich durch Veresterung von Alkoholen entsprechend der Formel I, worin A ein Cyclohexanring ist, aber an Stelle mindestens eines der Reste $X^1$ bis $X^6$ eine OH-Gruppe steht, mit Carbonsäuren der Formel R-COOH.

An Stelle der Carbonsäuren und/oder der Alkohole können auch ihre reaktionsfähigen Derivate verwendet werden.

Als reaktionsfähige Derivate der genannten Carbonsäuren eignen sich insbesondere die Säurehalogenide, vor allem die Chloride und Bromide, ferner die Anhydrlde, z. B. auch gemischte Anhydride, Azide oder Ester, insbesondere Alkylester mit 1 - 4 C-Atomen in der Alkylgruppe.

Als reaktionsfähige Derivate der genannten Alkohole kommen insbesondere die entsprechenden Metallalkoholate in Betracht, worin an Stelle der OH-Gruppe(n) OM-Gruppe(n) stehen und worin M ein aquivalent eines Metalls, vorzugsweise eines Alkalimetalls wie Na oder K, bedeutet.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid (HMPT), Kohlenwasserstoffe wie Benzol, Toluol oder Xylol, Halogenkohlenwasserstoffe wie Tetrachlorkohlenstoff oder Tetrachlorethylen, Sulfoxide wie Dimethylsulfoxid oder Sulfolan und Carbonsäuren wie Trifluoressigsäure. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z. B. Pyridin, Chinolin oder Triethylamin als Lösungsmittel für die Veresterung angewandt werden. Die Veresterung kann auch in Abwesenheit eines Lösungsmittels, z. B. durch einfaches Erhitzen der Komponenten in Gegenwart von Natriumacetat, durchgeführt werden. Die Reaktionstemperatur liegt gewöhnlich zwischen -50° und +250°, vorzugsweise zwischen -20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol zunächst in das Natrium- oder Kaliumalkoholat überführt, z. B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Saurechlorids oder Anhydrids in Diethylether, Aceton oder DMF versetzt, zweckmäßig bei Temperaturen zwischen etwa -25° und +20°

Sulfoxide und Sulfone der Formel I, worin mindestens einer der Reste $X^1$ bis $X^6$ -SOR oder -$SO_2R$ bedeutet, können durch Oxydation entsprechender Thioether der Formel I, worin mindestens einer der Reste $X^1$ bis $X^6$ -SR bedeutet, hergestellt, werden.

Man oxidiert je nach dem gewählten Reagenz und den angewendeten Bedingungen zu den entsprechenden Sulfoxiden (eine der Gruppen $X^1$ bis $X^6$ = SO) oder zu den entsprechenden Sulfonen (eine der Gruppen $X^1$ bis $X^6$ = $SO_2$), wobei man nach an sich aus der Literatur bekannten Methoden arbeitet und die Reaktionsbedingungen im einzelnen aus der Literatur leicht entnehmen kann. Will man die Sulfoxide erhalten, so oxydiert man beispielsweise mit Wasserstoffperoxid, Persäuren, Cr(VI)-Verbindungen wie Chromsäure, Salpetersäure, nitrosen Gasen, $N_2O_3$, Halogenen wie Chlor, Hypochloriten, $KMnO_4$, N-Bromsuccinimid, 1-Chlorbenztriazol, Ce(IV)-Verbindungen wie $(NH_4)\ Ce(NO_3)_6$, negativ substituierten aromatischen Diazoniumsalzen wie o- oder p-Nitrophenyldiazoniumchlorid oder elektrolytisch unter verhältnismäßig milden Bedingungen und bei relativ niedrigen Temperaturen (etwa -80 bis +100°). Will man dagegen die Sulfone erhalten, so verwendet man die gleichen Oxydationsmittel unter kräftigeren Bedingungen und/oder im Überschuß sowie in der Regel bei höheren Temperaturen. Bei diesen Umsetzungen können die üblichen inerten Lösungsmittel zugegen oder abwesend sein. Als inerte Lösungsmittel eignen sich beispielsweise Wasser, wässerige Mineralsäuren, wässerige Alkalilaugen, niedere Alkohole wie Methanol oder Ethanol, Ester wie Ethylacetat, Ketone wie Aceton, niedere Carbonsäuren wie Essigsäure, Nitrile wie Acetonitril, Kohlenwasserstoffe wie Benzol, chlorierte Kohlenwasserstoffe wie Chloroform oder $CCl_4$.

Ein bevorzugtes Oxidationsmittel ist 30 %iges wässeriges Wasserstoffperoxid. Dieses führt bei Anwendung der berechneten Menge in Lösungsmitteln wie Essigsäure, Aceton, Ethanol oder wässeriger Natronlauge bei

Temperaturen zwischen -20 und 100° zu den Sulfoxiden, im Überschuß bei höheren Temperaturen, vorzugsweise in Essigsäure oder in einem Gemisch aus Essigsäure und Acetanhydrid, zu den Sulfonen.

Ein weiteres bevorzugtes Oxidationsmittel ist 3-Chlorperbenzoesäure. Diese führt bei Anwendung der berechneten Menge in Lösungsmitteln wie Halogenkohlenwasserstoffen bei Temperaturen innerhalb von 0° zu den Sulfoxiden, im Überschuß bei Temperaturen zwischen 0° und Raumtemperatur zu den Sulfonen.

Eine weitere Möglichkeit zur Herstellung der Sulfoxide besteht darin, daß man die Thioether mit Chlor, z. B. in feuchtem Benzol oder in Essigsäure, behandelt. Die intermediär erhaltenen Dichlorverbindungen werden durch Hydrolyse sehr leicht in die Sulfoxide umgewandelt.

Die erfindungsgemäßen diskotischen flüssigkristallinen Phasen bestehen aus mindestens zwei, vorzugsweise 3 bis 12 Komponenten, darunter mindestens einer Verbindung der Formel I. Die anderen Bestandteile werden vorzugsweise ausgewählt aus den bekannten diskotischen flüssigkristallinen Substanzen, insbesondere aus den Klassen der hexasubstituierten Benzol- oder Triphenylen-Derivete. Die erfindungsgemäßen Phasen enthalten etwa 0,1 bis 100, vorzugsweise 10 bis 100 %, einer oder mehrerer Verbindungen der Formel I.

Die Herstellung der erfindungsgemäßen diskotischen flüssigkristallinen Phasen erfolgt in an sich üblicher Weise. In der Regel werden die Komponenten ineinander gelöst, zweckmäßig bei erhöhter Temperatur.

Die erfindungsgemäßen diskotischen flüssigkristellinen Phasen können weiterhin durch geeignete Zusätze modifiziert werden. Beispielsweise können Leitsalze zur Verbesserung der Leitfähigkeit, pleochroitische Farbstoffe oder Substenzen zur Veränderung der dielektrischen Anisotropie, der Viskosität und/oder der Orientierung der diskotischen Phasen zugesetzt werden.

Die folgenden Beispiele sollen die Erfindung erläutern, ohne sie zu begrenzen. F = Schmelzpunkt, K = Klärpunkt. Vor- und nachstehend bedeuten Prozentangaben Gewichtsprozent; alle Tempereturen sind in Grad Celsius angegeben. "Übliche Aufarbeitung" bedeutet: man gibt wesser hinzu, extrahiert mit Methylenchlorid, trennt ab, trocknet die organische Phase, dampft ein und reinigt das Produkt durch Kristallisation und/oder Chromatographie.

**Beispiel 1**

Ein Gemisch von 17,4 g Hexahydroxybenzol, 150 g Hexyljodid, 41,4 g $K_2CO_3$ und 250 ml DMF wird unter Rühren 16 Stunden auf 80° hitzt, dann abgekühlt und wie üblich aufgearbeitet. Man erhält Hexekis-(hexoxy)-benzol.

Analog erhält man durch Veretherung von scyllo-Inosit:

scyllo-Inosit-hexakis-(propylether)
scyllo-Inosit-hexakis-(butylether)
scyllo-Inosit-hexakis-(pentylether)
scyllo-Inosit-hexakis-(hexylether)
scyllo-Inosit-hexakis-(heptylether)
scyllo-Inosit-hexakis-(octylether)
scyllo-Inosit-hexakis-(nonylether)
scyllo-Inosit-hexakis-(decylether)
scyllo-Inosit-hexakis-(undecylether)
scyllo-Inosit-hexakis-(trideciIether)
scyllo-Inosit-hexakis-(tetradecylether)
scyllo-Inosit-hexakis-(pentadecylether).

**Beispiel 2**

Ein Gemisch von 1,8 g scyllo-Inosit, 12 g Octanoylchlorid und 20 ml Trifluoressigsäure wird 2 Stunden bei 20° gerührt. Nach dem Eindampfen und üblicher aufarbeitung (Elution mit Hexan-Ethylacetat 20:1) erhält man Hexakis-(octanoyl)-scyllo-inosit, F. 77,5°; K. 198,4°.

Analog erhält man mit den entsprechenden Säurechloriden:

Hexaacetyl-scyllo-inosit
Hexapropionyl-scyllo-inosit
Hexabutyryl-scyllo-inosit
Hexaisobutyryl-scyllo-inosit
Hexavaleryl-scyllo-inosit
Hexacapronyl-scyllo-inosit
Hexkis-(heptanoyl)-scyllo-inosit
Hexakis-(nonanoyl)-scyllo-inosit
Hexakis-(decanoyl)-scyllo-inosit
Hexakis-(undecanoyl)-scyllo-inosit
Hexakis-(dodecanoyl)-scyllo-inosit

Hexakis-(tridecanoyl)-scyllo-inosit
Hexakis-(tetradecanoyl)-scyllo-inosit
Hexekis-(pentadecanoyl)-scyllo-inosit.

**Beispiel 3**

Zu einer Lösung von 10,26 g Hexakis-(nonylthio)-benzol in 400 ml Essigsäure tropft man innerhalb 30 Minuten unter Rühren bei 75 - 80° eine Lösung von 6,42 ml 30 %igem $H_2O_2$ in 150 ml Essigsäure. Man hält 2 Stunden bei 80°, kocht auf, gießt in Wasser, arbeitet wie üblich auf und erhält Hexakis-(nonylsulfinyl)-benzol.

Analog erhält man durch Oxydation der entsprechenden Thioether:
Hexakis-(methylsulfinyl)-benzol
Hexakis-(ethylsulfinyl)-benzol
Hexakis-(propylsulfinyl)-benzol
Hexakis-(butylsulfinyl)-benzol
Hexakis-(pentylsulfinyl)-benzol
Hexakis-(hexylsulfinyl)-benzol
Hexakis-(heptylsulfinyl)-benzol
Hexakis-(octylsulfinyl)-benzol
Hexakis-(decylsulfinyl)-benzol
Hexakis-(undecylsulfinyl)-benzol
Hexakis-(dodecylsulfinyl)-benzol
Hexakis-(tridecylsulfinyl)-benzol
Hexakis-(tetradecylsulfinyl)-benzol
Hexekis-(pentadecylsulfinyl)-benzol
scyllo-1,2,3,4,5,6-Hexakis-(methylsulfinyl)-cyclohexan
scyllo-1,2,3,4,5,6-Hexakis-(ethylsulfinyl)-cyclohexan
scyllo-1,2,3,4,5,6-Hexakis-(propylsulfinyl)-cyclohexen
scyllo-1,2,3,4,5,6-Hexakis-(butylsulfinyl)-cyclohexan
scyllo-1,2,3,4,5,6-Hexakis-(pentylsulfinyl)-cyclohexan
scyllo-1,2,3,4,5,6-Hexakis-(hexylsulfinyl)-cyclohexan
scyllo-1,2,3,4,5,6-Hexakis-(heptylsulfinyl)-cyclohexan
scyllo-1,2,3,4,5,6-Hexakis-(octylsulfinyl)-cyclohexan
scyllo-1,2,3,4,5,6-Hexakis-(nonylsulfinyl)-cyclohexan
scyllo-1,2,3,4,5,6-Hexakis-(decylsulfinyl)-cyclohexen
scyllo-1,2,3,4,5,6-Hexakis-(undecylsulfinyl)-cyclohexan
scyllo-1,2,3,4,5,6-Hexakis-(dodecylsulfinyl)-cyclohexan
scyllo-1,2,3,4,5,6-Hexakis-(tridecylsulfinyl)-cyclohexan
scyllo-1,2,3,4,5,6-Hexakis-(tetradecylsulfinyl)-cyclohexan
scyllo-1,2,3,4,3,6-Hexakis-(pentadecylsulfinyl)-cyclohexan.

**Beispiel 4**

Man kocht 10,26 g Hexakis-(nonylthio)-benzol mit einem 20 %-igen Überschuß an 85 - 90-%iger m-Chlorperbeizoesäure in insgesamt 60 ml $CHCl_3$ 48 Stunden, dampft ein, kristallisiert aus Ethanol/Petrolether um und erhält Hexakis- (nonylsulfonyl)-benzol, F. 95°, K. 131°.

Analog erhält man durch Oxydation der entsprechenden Thioether:
Hexakis-(methylsulfonyl)-benzol
Hexakis-(ethylsulfonyl)-benzol
Hexakis-(propylsulfonyl)-benzol
Hexakis-(butylsulfonyl)-benzol
Hexakis-(pentylsulfonyl)-benzol
Hexakis-(hexylsulfonyl)-benzol
Hexakis-(heptylsulfonyl)-benzol
Hexakis-(octylsulfonyl)-benzol
Hexakis-(decylsulfonyl)-benzol
Hexakis-(undecylsulfonyl)-benzol, F. 76°, K. 116°
Hexakis-(dodecylsulfonyl)-benzol
Hexakis-(tridecylsulfonyl)-benzol, F. 60°, K. 89°
Hexakis-(tetradecylsulfonyl)-benzol
Hexakis-(pentadecylsulfonyl)-benzol
scyllo-1,2,3,4,5,6-Hexakis-(methylsulfonyl)-cyclohexan

scyllo-1,2,3,4,5,6-Hexakis-(ethylsulfonyl)-cyclohexan
scyllo-1,2,3,4,5,6-Hexakis-(propylsulfonyl)-cyclohexan
scyllo-1,2,3,4,5,6-Hexakis-(butylsulfonyl)-cyclohexan
scyllo-1,2,3,4,5,6-Hexakis-(pentylsulfonyl)-cyclohexan
scyllo-1,2,3,4,5,6-Hexakis-(hexylsulfonyl)-cyclohexan
scyllo-1,2,3,4,5,6-Hexakis-(heptylsulfonyl)-cyclohexan
scyllo-1,2,3,4,5,6-Hexakis-(octylsulfonyl)-cyclohexan
scyllo-1,2,3,4,5,6-Hexakis-(nonylsulfonyl)-cyclohexan
scyllo-1,2,3,4,5,6-Hexakis-(decylsulfonyl)-cyclohexan
scyllo-1,2,3,4,5,6-Hexakis-(undecylsulfonyl)-cyclohexan
scyllo-1,2,3,4,5,6-Hexakis-(dodecylsulfonyl)-cyclohexan
scyllo-1,2,3,4,5,6-Hexakis-(tridecylsulfonyl)-cyclohexan
scyllo-1,2,3,4,5,6-Hexakis-(tetradecylsulfonyl)-cyclohexan
scyllo-1,2,3,4,5,6-Hexakis-(pentadecylsulfonyl)-cyclohexan.
1,3,5-Tris-(propylsulfonyl)-benzol
1,3,5-Tris-(butylsulfonyl)-benzol
1,3,5-Tris-(pentylsulfonyl)-benzol
1,3,5-Tris-(hexylsulfonyl)-benzol
1,3,5-Tris-(heptylsulfonyl)-benzol
1,3,5-Tris-(octylsulfonyl)-benzol
1,3,5-Tris-(nonylsulfonyl)-benzol
1,3,5-Tris-(decylsulfonyl)-benzol
1,3,5-Tris-(undecylsulfonyl)-benzol
1,3,5-Tris-(dodecylsulfonyl)-benzol
1,3,5-Tris-(tridecylsulfonyl)-benzol
1,3,5-Tris-(tetradecylsulfonyl)-benzol
1,3,5-Tris-(pentadecylsulfonyl)-benzol
1,2,3,5-Tetrakis-(propylsulfonyl)-benzol
1,2,3,5-Tetrakis-(butylsulfonyl)-benzol
1,2,3,5-Tetrakis-(pentylsulfonyl)-benzol
1,2,3,5-Tetrakis-(hexylsulfonyl)-benzol
1,2,3,5-Tetrakis-(heptylsulfonyl)-benzol
1,2,3,5-Tetrakis-(octylsulfonyl)-benzol
1,2,5,5-Tetrakis-(nonylsulfonyl)-benzol
1,2,3,5-Tetrakis-(decylsulfonyl)-benzol
1,2,3,5-Tetrakis-(undecylsulfonyl)-benzol
1,2,3,5-Tetrakis-(dodecylsulfonyl)-benzol
1,2,3,5-Tetrakis-(tridecylsulfonyl)-benzol
1,2,3,5-Tetrakis-(tetradecylsulfonyl)-benzol
1,2,3,5-Tetrikis-(pentadecylsulfonyl)-benzol
r-1, c-3, c-5-Tris-(propylsulfonyl)-cyclohexan
r-1, c-3, c-5-Tris-(butylsulfonyl)-cyclohexan
r-1, c-3, c-5-Tris-(pentylsulfonyl)-cyclohexan
r-1, c-3, c-5-Tris-(hexylsulfonyl)-cyclohexan
r-1, c-3, c-5-Tris-(heptylsulfonyl)-cyclohexan
r-1, c-3, c-5-Tris-(octylsulfonyl)-cyclohexan
r-1, c-3, c-5-Tris-(nonylsulonyl)-cyclohexan
r-1, c-3, c-5-Tris-(decylsulfonyl)-cyclohexan
r-1, c-3, c-5-Tris-(undecylsulfonyl)-cyclohexan
r-1, c-3, c-5-Tris-(dodecylsulfonyl)-cyclohexan
r-1, c-3, c-5-Tris-(tridecylsulfonyl)-cyclohexan
r-1, c-3, c-5-Tris-(tetradecylsulfonyl)-cyclohexan
r-1, c-3, c-5-Tris-(pentadecylsulfonyl)-cyclohexan.

**Beispiel 5**

Ein Gemisch von 0,9 g scyllo-Inosit-pentakis-(nonylether), 0,2 g Octanoylchlorid und 10 ml Trifluoressigsäure wird 2 Stunden bei Raumtemperatur gerührt. Nach dem Eindampfen und üblicher Auferbeitung erhält man Octanoyl-scyllo-inosit-pentakis-(nonylether).

Analog erhält man mit den entsprechenden Säurechloriden:
Propionyl-scyllo-inosit-pentekis-(nonylether)
Butyryl-scyllo-inosit-pentakis-(nonylether)
Veleryl-scyllo-inosit-pentakis-(nonylether)

Capronyl-scyllo-inosit-pentakis-(nonylether)
Heptanoyl-scyllo-inosit-pentakis-(nonylether)
Nonanoyl-scyllo-inosit-pentakis-(nonylether)
Undecanoyl-scyllo-inosit-pentakis-(nonylether)
Dodecanoyl-scyllo-inosit-pentakis-(nonylether)
Tridecanoyl-scyllo-inosit-pentakis-(nonylether)
Tetradecanoyl-scyllo-inosit-pentakis-(nonylether)
Pentadecanoyl-scyllo-inosit-pentakis-(nonylether).

**Patentansprüche**

1. Sechsringverbindungen der Formel:

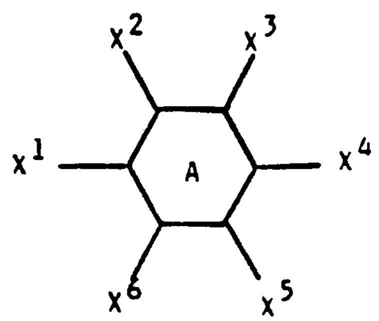

I

worin
A ein Benzol- oder Cyclohexanring ist,
$X^6$ bis $X^6$ jeweils unabhängig voneinander H, -SOR, $-SO_2R$ oder, falls A ein Cyclohexanring ist, auch -OR oder -O-COR,
bedeuten, und
R jeweils eine Alkylgruppe mit bis zu 15 C-Atomen, worin eine oder zwei $CH_2$ -Gruppen durch O-Atome ersetzt sein können, ist,
wobei mindestens drei der Substituenten $X^1$ bis $X^6$ von H verschieden sind, und falls A einen Cyclohexanring bedeutet, die jeweils gegenüberliegenden Substituenten equatorial und in trans-Stellung zueinander stehen.

2. a) Hexaoctanoyl-scyllo-inosit, gemäß Anspurch 1.
b) Hexakis-(nonylsulfonyl)-benzol, gemäß Anspurch 1.
c) Hexakis-(undecylsulfonyl)-benzol, gemäß Anspruch 1.
d) Hexakis-(tridecylsulfonyl)-benzol, gemäß Anspruch 1.

3. Verfahren zur Herstellung von Verbindungen der Formel I nach Anspruch 1, dadurch gekennzeichnet, daß man zur Herstellung von Ethern der Formel I, worin mindestens einer der Reste $X^1$ bis $X^6$ -OR bedeutet, eine entsprechende Hydroxyverbindung verethert
und/oder daß man zur Herstellung von Estern der Formel I, worin mindestens einer der Reste $X^1$ bis $X^6$ -O-COR bedeutet, eine entsprechende Carbonsäure verestert
und/oder daß man zur Herstellung von Sulfoxiden der Formel I, worin mindestens einer der Reste $X^1$ bis $X^6$ -SOR bedeutet, einen entsprechenden Thioether oxidiert
und/oder daß man zur Herstellung von Sulfonen der Formel I, worin mindestens einer der Reste $X^1$ bis $X^6$ -SOR bedeutet, einen entsprechenden Thioether oder ein entsprechendes Sulfoxid oxydiert.

4. Verwendung der Verbindungen der Formel I nach Anspruch 1 als Komponenten diskotischer flussigkristallinar Phasen.

5. Diskotische flüssigkristalline Phasen mit mindestens zwei flüssigkristallinen Komponenten, dadurch gekennzeichnet, daß mindestens eine Komponente eine Verbindung der Formel I nach Anspruch 1 ist.

6. Flüssigkristall-Anzeigeelement, dadurch gekennzeichnet, daß es eine flüssigkristalline Phase nach Anspruch 5 enthält.

## Claims

1. Six-membered ring compounds of the formula I:

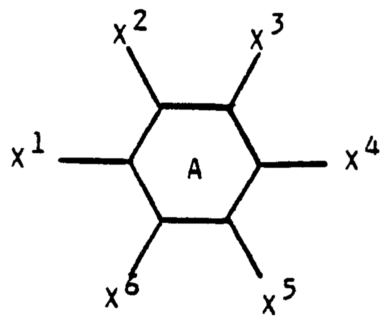

wherein
A is a benzene or cyclohexane ring
$X^1$ to $X^6$ independently of one another in each case
$X^1$ to $X^6$ independently of one another in each case are H, -SOR, $-SO_2R$ or, if A is a cyclohexane ring, also OR or -O-COR, and
R is in each case an alkyl group which has up to 15 C atoms and in which one or two $CH_2$ groups can be replaced by 0 atoms, at least three of the substituents $X^1$ to $X^6$ being other than H, and, if A is a cyclohexane ring, in each case the opposite substituents are equatorial and in trans position to each other.

2. a) Hexaoctanoylscyllo-inositol, according to Claim 1.
b) Hexakis-(nonylsulfonyl)-benzene, according to Claim 1.
c) Hexakis-(undecylsulfonyl)-benzene, according to Claim 1.
d) Hexakis-(tridecylsulfonyl)-benzene, according to Claim 1.

3. Process for the preparation of compounds of the formula I according to Claim 1, characterised in that ethers of the formula I wherein at least one of the radicals $X^1$ to $X^6$ is -OR are prepared by etherifying a corresponding hydroxy compound, and/or esters of the formula I wherein at least one of the radicals $X^1$ to $X^6$ corresponding hydroxy compound, and/or esters of the formula I wherein at least one of the radicals $X^1$ to $X^6$ is -O-COR are prepared by esterifying a corresponding carboxylic acid, and/ or sulfoxides of the formula wherein at least one of the radicals $X^1$ to $X^6$ is -SOR are prepared by oxidising a corresponding thioether, and/or sulfones of the formula I wherein at least one of the radicals $X^1$ to $X^6$ is $-SO_2R$ are prepared by oxidising a corresponding thioether or a corresponding sulfoxide.

4. The use of the compounds of the formula I according to Claim 1 as components of discotic, liquid-crystal phases.

5. Discotic, liquid-crystal phases containing at least two liquid-crystal components, characterised in that at least one component is a compound of the formula I according to Claim 1.

6. Liquid-crystal display element characterised in that it contains a liquid-crystal phase according to Claim 5.

## Revendications

1. Composés à noyau hexagonal de formule I :

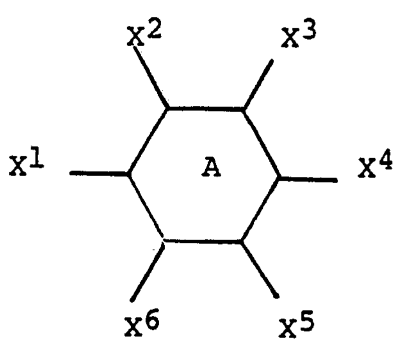

I

dans laquelle
A est un noyau benzène ou cyclohexane,
$X^1$ à $X^6$ signifient indépendamment chaque fois les uns des autres H, -SOR, $-SO_2R$ ou bien, lorsque A est un noyau cyclohexane, également -OR ou -O-COR,
et
R étant chaque fois un groupe alcoyle ayant jusqu'à 15 atomes de carbone et un ou deux groupes $CH_2$

pouvant être remplacés par des atomes O,

au moins trois des substituants $X^1$ à $X^6$ étant différents de H et, au cas où A signifie un noyau cyclohexane, les substituants chaque fois en opposition étant mutuellement équatoriaux et en position trans.

2. a) Hexaoctanoyl-scyllo-inositol selon la revendication 1.

b) hexakis-(nonylsulfonyl)-benzène selon la revendication 1.

c) hexakis-(undécylsulfonyl)-benzène selon la revendication 1.

d) hexakis-(tridécylsulfonyl)-benzène selon la revendication 1.

3. Procédé de fabrication de composés de formule I selon la revendication 1, caractérisé en ce que pour la fabrication d'éthers de formule I, où au moins un des radicaux $X^1$ à $X^6$ signifie -OR, on éthérifie un composé hydroxylé correspondant, et/ou en ce que pour la fabrication d'esters de formule I, où au moins un des radicaux $X^1$ à $X^6$ signifie -O-COR, on estérifie un acide carboxylique correspondant,

et/ou en ce que pour la fabrication de sulfoxydes de formule I, où au moins un des radicaux $X^1$ à $X^6$ signifie -SOR, on oxyde un thioéther correspondant, et/ou en ce que pour la fabrication de sulfones de formule I , où au moins un des radicaux $X^1$ à $X^6$ signifie $-SO_2R$, on oxyde un thioéther correspondant ou un sulfoxyde correspondant.

4. Utilisation des composés de formule I selon la revendication 1 comme composants de phases cristallines liquides discotiques.

5. Phases cristallines liquides discotiques avec au moins deux composants cristallins liquides, caractérisées en ce qu'au moins un composant est un composé de formule I selon la revendication 1.

6. Èlément indicateur cristallin liquide, caractérisé en ce qu'il contient une phase cristalline liquide selon la revendication 5.